# EUROPEAN PATENT SPECIFICATION

(11) **EP 0 684 222 B1**
(45) Date of publication and mention of the grant of the patent: **14.10.1998**
(21) Application number: 95303508.6
(22) Date of filing: 24.05.1995
(51) Int. Cl.: C07C 69/653, C07C 69/76, A61K 6/02

(54) **Novel (meth)acrylate monomers and denture base compositions prepared therefrom**
Monomere (Meth)acrylate und damit hergestellte künstliche Gebisskompositionen
(Méth)acrylates monomères et compositions de prothèses dentaires préparées de ceux-ci

(30) Priority: 26.05.1994 GB 9410578
(43) Date of publication of application: 29.11.1995
(73) Proprietor: Abonetics Limited, London E1 4NS (GB)
(72) Inventor: Davy, Kenneth Walter Michael, Midhurst, West Sussex, GU29 9TB (GB)
(74) Representative: Allard, Susan Joyce

(56) References cited:
- DE-A- 2 647 890
- US-A- 3 346 620

## Description

The present invention relates to X-ray opaque compositions comprising (meth)acrylate monomers and, in particular, to X-ray opaque denture base compositions prepared therefrom.

Denture base compositions have previously been prepared by mixing approximately two-thirds of polymethylmethacrylate beads with one third of methyl methacrylate monomer, packing the dough so formed into-plaster moulds and heat curing. Polymethylmethacrylate is however, a brittle material and many difficulties have been caused since its introduction as a denture base composition, since fragments have been aspirated, swallowed or impacted into the body during traumatic accidents, or through accidental breakage during use. The polymer is transparent to X-rays and fragments are difficult to detect and only can be located when clinical complications arising from their presence become apparent.

Attempts to remedy this deficiency include the incorporation into the compositions of X-ray opaque glasses, inorganic fillers or simple chemical additives containing halogen. None of these approaches has been really successful since halogen containing chemicals leach out and barium containing glasses, at the concentrations necessary to give good X-ray opacity, result in poor adhesion of acrylic teeth to the denture.

The only X-ray opaque material that is currently used in denture base compositions is barium sulfate which is generally incorporated in an amount of up to 8% by weight of the polymethylmethacrylate beads. This is the maximum amount of the additive which can be included in the composition before the mechanical properties deteriorate. The incorporation of up to 8% of barium sulfate into the dental base composition does not provide sufficient opacity where the denture may be as thin as 2mm, and, furthermore, the cured denture base composition containing barium sulfate has a chalky appearance and not the gum-like translucency of standard denture base compositions.

We have now developed a range of (meth)acrylate monomers which exhibit X-ray opacity and which can be incorporated into denture base compositions based on polymethylmethacrylate.

Accordingly, the present invention provides an X-ray opaque composition which comprises an acrylate or methacrylate monomer having the general formula 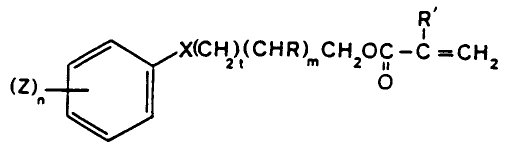
where X is -O- 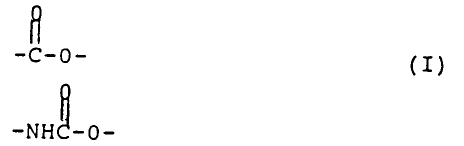
R is H,OH,CH₃, or 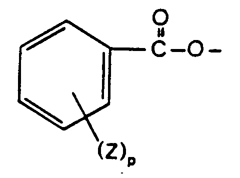
R' is H or CH₃
Z is Br or I, or a mixture of these substituents
m is 0 or 1
n is 3 to 5
p is 3 to 5
and t is 0 or 1,
with the proviso that when X is 0 then R is OH or 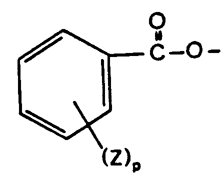
and m is 1; or a polymer or copolymer prepared therefrom.

It will be understood that the acrylate or methacrylate monomers used in the present invention may contain an asymmetric carbon atom and it is intended that the different isomers of the monomers are included with the scope of the present invention, as well as the racemic mixtures thereof.

In the above Formula the group Z may be bromine or iodine, or a mixture of bromine and iodine substituents may be used. Preferably, however, Z is iodine since the monomers containing iodine will generally have a greater X-ray opacity than those containing bromine. The integers n and p are preferably 3.

Methacrylate iodine-containing monomers falling within the scope of the invention include the monomers of the general formulae:

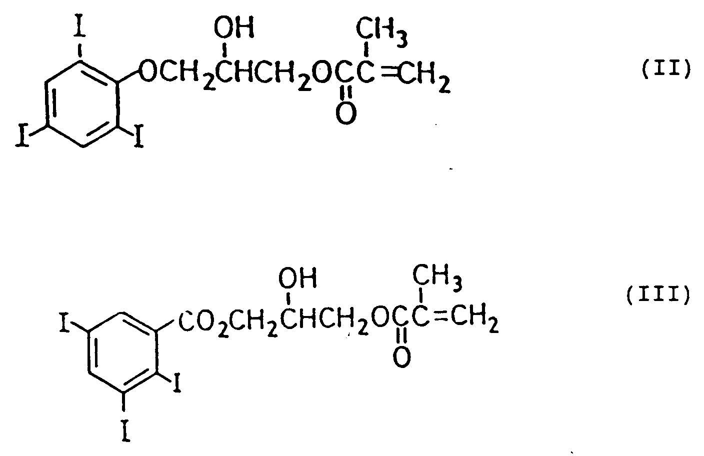

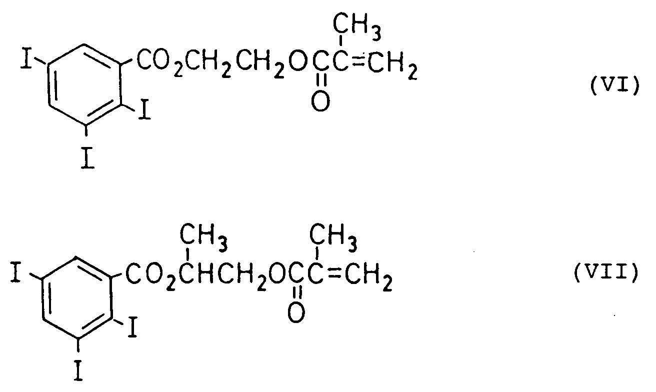

The compounds of Formula (I) in which R is OH, m is 1, t is 1 and R',X, Z, n and p are as defined above may be prepared by the following reaction scheme

### A.

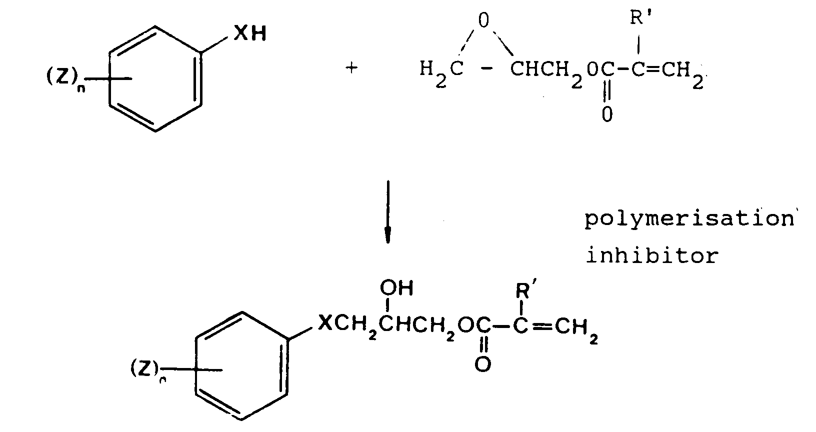

The compounds of Formula (I) in which X is 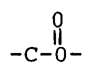 or 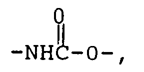 R is H or CH₃ and R', **Z**, m, n, p and t are as defined above may be prepared by the following reaction scheme B

### B

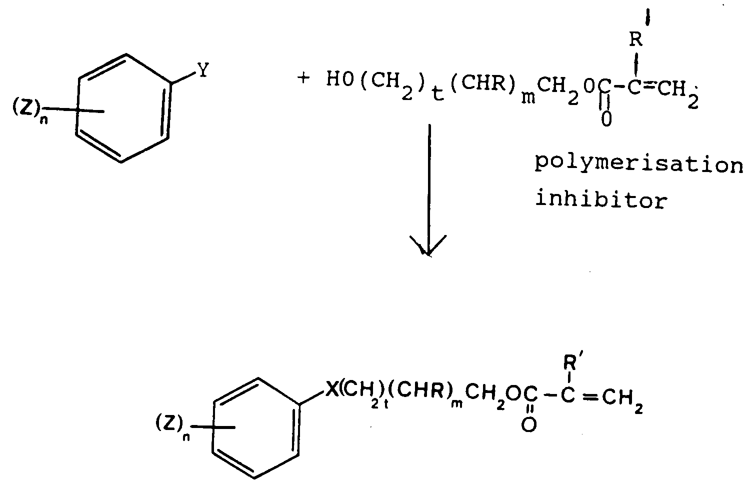

where Y is -COHal or -NCO where Hal is Cl or Br.

The compounds of formulae (VI) and (VII) may be prepared according to reaction Scheme B, as follows:

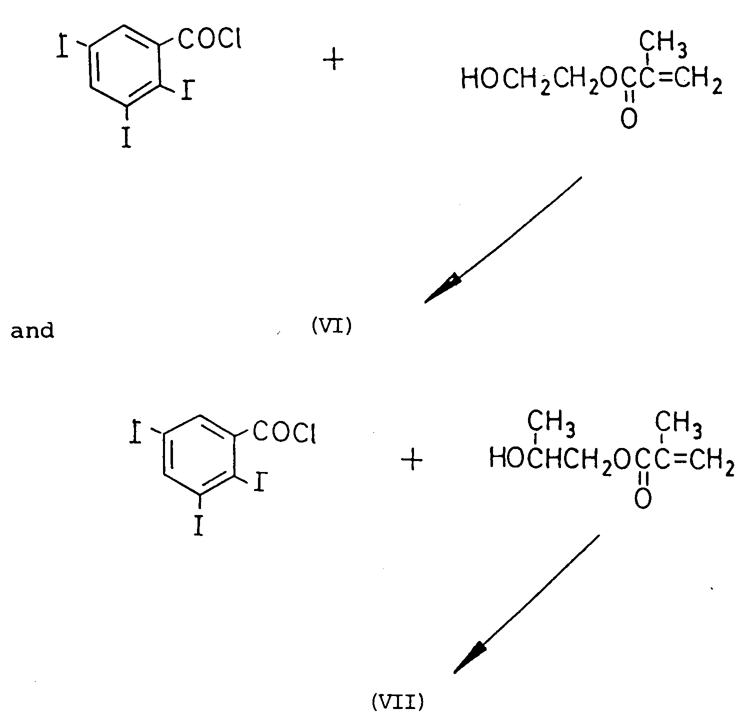

The compounds of Formulae (II) and (III) may be prepared as follows:

### C. Reaction of tri-iodophenol with glycidyl methacrylate

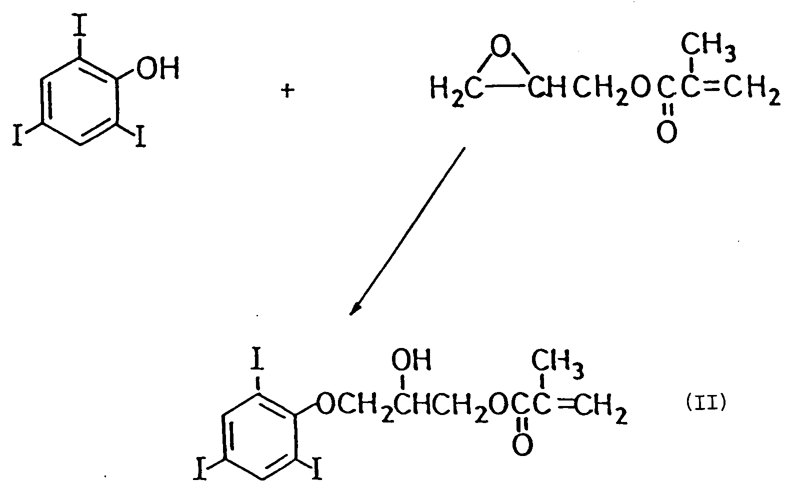

### D. Reaction of tri-iodobenzoic acid with glycidyl methacrylate

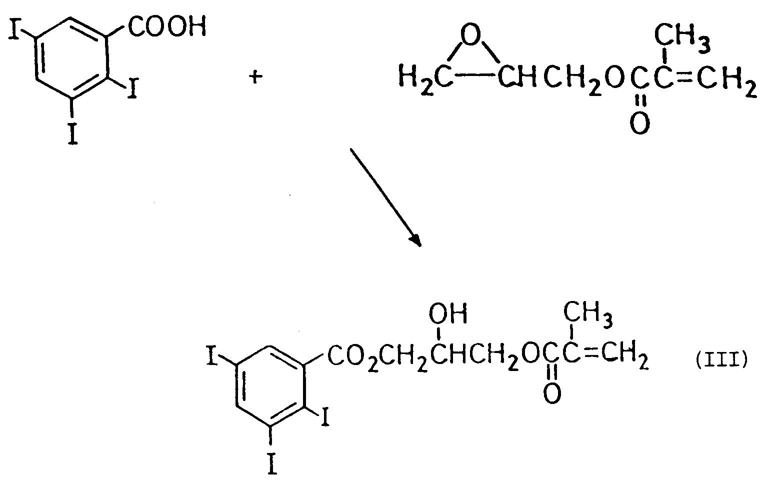

The compounds of Formula (I) in which R is 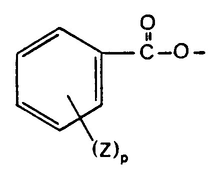 may be prepared by reacting a corresponding compound of Formula (I) in which R is an OH group with a compound of the formula: 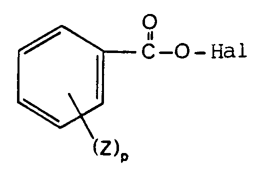
where Hal is Cl or Br.

For example the methacrylate monomers of the Formulae (II) and (III) may be converted into other monomers falling within the scope of the invention by reaction with tri-iodobenzoyl chloride according to the following reaction schemes, or by other methods of esterification such as described in Example 5:

### E

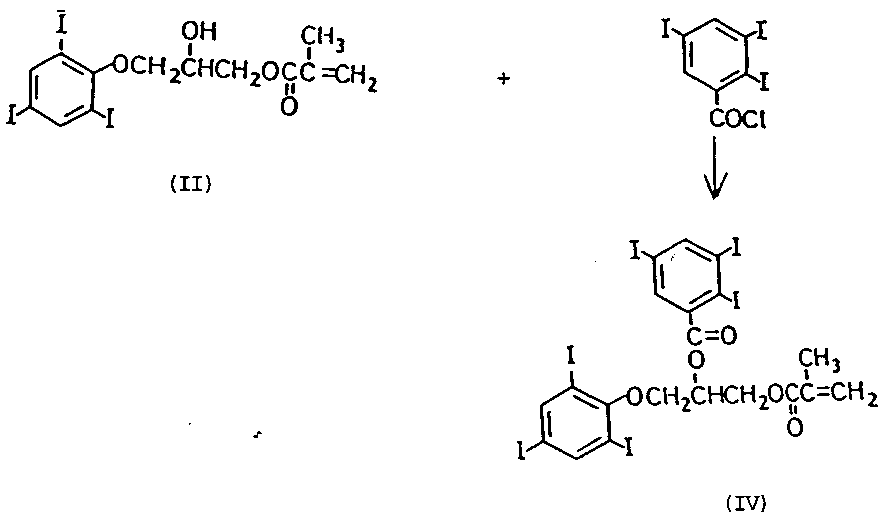

### F

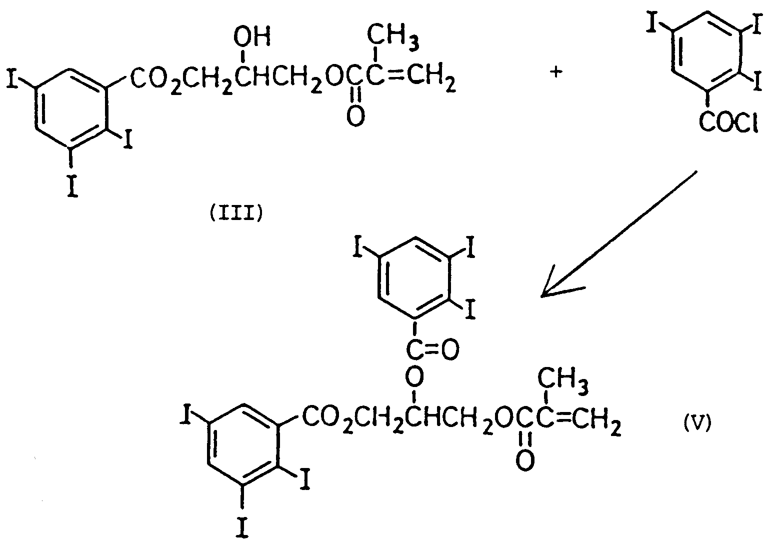

The acrylate or methacrylate monomers used in the present invention are of particular use in the preparation of X-ray opaque denture base compositions, preferably an X-ray opaque acrylic resin composition.

Acrylic resin denture base compositions are generally prepared by polymerising polymethylmethacrylate, in the form of beads, with a methyl methacrylate monomer to form a dough which is then moulded to the desired shape and cured. The curing is carried out by methods generally known in the art, for example heat curing in the presence of a suitable catalyst such as benzoyl peroxide, or cold curing in the presence of peroxide/amine catalyst systems.

The acrylate or methacrylate monomers used in the present invention may be incorporated by copolymerization into the polymethylmethacrylate beads, or may replace at least a portion of the methyl methacrylate monomer. The polymethylmethacrylate beads used in the denture base resins generally have a size in the range of from 40 to 250µm, preferably less than 150µm.

Generally, the acrylate or methacrylate monomers used in the present invention are incorporated into the denture base compositions, either as the monomers or as the polymers prepared from the monomers, in an amount of from 5 to 50% by weight.

The acrylate or methacrylate monomers used in the present invention may also be used to prepare X-ray opaque rubbers or X-ray opaque copolymer beads by copolymerisation with at least one monomer such as methyl methacrylate using conditions known in the art.

Furthermore, the acrylate or methacrylate monomers used in the present invention may be used in the preparation of X-ray opaque bone cements as a replacement for the barium sulfate currently used in such compositions.

Another use of the acrylate or methacrylate monomers of the present invention is in the formulation of radioopaque coatings for the coating of various medical inserts, such as coronary guidewires, and medical implants such as stents.

A still further use of the acrylate or methacrylate monomeric compositions of the present invention is for use as a standard for the mineral quantitation of human bones by backscattered electron imaging.

A further use of the acrylate or methacrylate polymeric compositions of the present invention is the formulation of optically clear polymer glasses with high refractive indices for use in contact lens formulations.

A still further use of the acrylate or methacrylate polymeric compositions of the present invention is in the formulation of radiopaque acrylic teeth for use in the X-ray opaque denture base formulation described herein or in a standard radiolucent polymethylmethacrylate denture base.

A particular advantage of the acrylate or methacrylate polymers used in the present invention relates to the processing of the polymer. With a glass transition temperature of about 100°C and neglible thermal decomposition below 325°C, injection moulding or other polymer moulding techniques that rely upon plastic flow at elevated temperatures are possible, thereby increasing the versatility of the polymer.

Another advantage of the acrylate or methacrylate polymers used in the present invention relates to the flame retarding nature of those polymers that contain 3 or more iodine atoms.

The present invention will be further described with reference to the following Examples.

### EXAMPLE 1

### Preparation of 2-Hydroxy-3-(2,4,6-triodophenoxy) propylmethacrylate

0.1 mol of 2,4,6-tri-iodophenol was stirred with 0.1 mol of glycidyl methacrylate at 65°C in the presence of 1 to 2% hydroquinone polymerisation inhibitor and 1 to 2% of N,N'-dimethyl-p-toluidine. After 5 hours the liquid was dissolved in ethyl acetate and extracted repeatedly with dilute HCl (0.2M) and then with saturated sodium bicarbonate solution. The organic phase was dried (MgSO₄) and the solvent was removed under reduced pressure and the resulting crystalline solid recrystallised three times from diethyl ether to give as a white solid,2-hydroxy-3-(2,4,6-triodophenoxy)-propylmethacrylate m.pt. 92°C; yield (90%).

| | | |
|---|---|---|
| Calculated for C₁₃H₁₃O₄I₃ : | C = 26.12%; | H = 2.17% |
| Found : | C = 26.05%; | H = 2.20% |

### EXAMPLE 2

### Preparation of 2-Hydroxy-3-(2,3,5-triiodobenzoyloxy)propylmethacrylate

100g (0.2 mol) of 2,3,5-tri-iodobenzoic acid was stirred with 28.4g (0.2 mol) of glycidyl methacrylate at 48-50°C in the presence of 4% w/w triethylamine. After 5 hours the liquid was dissolved in ethyl acetate and extracted repeatedly with dilute HCl (0.2M) and then with saturated sodium bicarbonate solution. The organic phase was dried (MgSO₄)and the solvent was removed under reduced pressure and the resulting yellow oil was passed through a short dry column of silica and charcoal to give 2-hydroxy-3-(2,3,5-triiodobenzoyloxy)propylmethacrylate as an almost colourless oil (yield 42.2g)

| | | |
|---|---|---|
| Calculated for C₁₄H₁₃O₅I₃ : | C = 26.20%; | H = 2.03% |
| Found : | C = 26.24%; | H = 2.55%. |

### EXAMPLE 3

### Preparation of 2,3-bis(2,3,5-triiodobenzoyloxy)propyl methacrylate

A solution of pyridine (6.0g) in dichloromethane (100 ml) was added to a stirred suspension of 2,3,5-triiodobenzoyl chloride (39.0g) in dichloromethane (300 ml) at 5°C over a period of 1 hour. This mixture was stirred for a further hour at 5°C and then a solution of 2-hydroxy-3-(2,3,5-triiodobenzoyloxy) propyl methacrylate (32.0g) in dichloromethane (200 ml) was added dropwise to the stirred suspension over a period of 3 hours whilst maintaining the temperature at 5°C. The mixture was then stirred overnight at room temperature (during which time the colour of the precipitate had changed from yellow to white). The precipitate was filtered off and washed with dichloromethane. The combined dichloromethane solution was extracted with dilute HCl (0.2M) and then with saturated sodium bicarbonate solution. The organic phase was dried (MgSO₄) and the solvent removed under reduced pressure to give 56.1g of a yellow oil. Flash chromatography on silica gel (eluted with pet.ether/ 40% dichloromethane) gave a crystalline solid (21.4g). Recrystallization from dichloromethane gave a pure material as white crystals, m.pt. 79°C (DSC).

| | | |
|---|---|---|
| Calculated for C₂₂H₁₄O₆I₆ : | C = 22.44%; | H = 1.25% |
| Found : | C = 23.29%; | H = 1.62% |

### EXAMPLE 4

### Preparation of 2-(2,3,5-triiodobenzoyloxy) ethyl methacrylate

2-hydroxyethyl methacrylate (13.0g) and pyridine (8.0g) were dissolved in dichloromethane (200 ml) and stirred at 5°C. A solution of 2,3,5-triiodobenzoyl chloride (50g) dissolved in dichloromethane (200 ml) was added dropwise over 3 hours whilst maintaining the temperature between 0-5°C. The mixture was stirred at room temperature overnight and the solid that had separated was filtered off and the organic phase was extracted first with dilute HCl (0.2M) and then with saturated sodium bicarbonate solution. The organic phase was dried (MgSO₄) and the solvent stripped off under reduced pressure yielding 40.4g of a yellow oil that crystallised on standing. Three recrystallizations from ethyl acetate gave pure material mpt. 94°C.

| | | |
|---|---|---|
| Calculated for C₁₃H₁₁O₄I₃ : | C = 25.54%; | H = 1.83% |
| Found : | C = 25.38%; | H = 1.88% |

### EXAMPLE 5

### Preparation of 2-(2,3,5-triiodobenzoyloxy)propyl methacrylate

2,3,5-triiodobenzoic acid (100g) was added over a period of 5 minutes to a stirred solution of 2-hydroxypropyl methacrylate (32.0g), 1,3-dicyclohexylcarbodiimide (46.0g) and 4-pyrrolidine pyridine (3.0g) in ether (1000 ml) at room temperature. The temperature of the mixture increased to 35°C during the addition and the mixture was stirred for 3 hours at room temperature. The solid was filtered off, the residue washed with fresh ether and the combined ether solution was extraced first with dilute HCl (0.2M) and then with saturated sodium bicarbonate solution. The ether was dried (MgSO₄) and the solvent removed under pressure to give 84.3g of a pale brown oil which crystallised on standing. Three crystallisations from ether gave a pure material m.pt 70°C (DSC).

| | | |
|---|---|---|
| Calculated, for C₁₄H₁₃O₄I₃ : | C = 26.87% | H = 2.08% |
| Found : | C = 26.78%; | H = 2.37% |

### EXAMPLE 6

### Copolymer beads of MMA and 2-hydroxy-3-(2,4,6-triiodophenoxy)propyl methacrylate

A solution of 2-hydroxy-3-(2,4,6-triiodophenoxy)propyl methacrylate (25g)in methyl methacrylate (75g) containing 2% w/w benzoyl peroxide was suspended in aqueous starch solution (60g in 600 ml) contained in a reaction vessel equipped with a thermometer, reflux condenser, nitrogen inlet and mechanical stirrer. The suspension was stirred vigorously under an atmosphere of nitrogen initially for one hour at 60°C, then over the course of the next three hours the temperature of the reaction flask was slowly raised at 90°C and held at this temperature for a further hour. The suspension was then poured into 5 litres of hot water, stirred and the copolymer beads allowed to settle out. The beads, which has an average diameter of 150µm, were washed twice by resuspending in hot water and allowing to settle out. Finally the beads were filtered off, washed with water and dried under vaccum at room temperature giving 63.2g of copolymer beads.

### EXAMPLES 7 and 8

### Sample preparation

22g of copolymer beads (average diameter 40-60µm) prepared from methyl methacrylate containing 25% w/w of the iodinated monomer of Example 4, or with 25% w/w of the iodinated monomer of Example 2, respectively, were mixed with 10 ml of methyl methacrylate monomer. Each of the resulting doughs containing about 16% by weight of the iodinated polymer was packed into plaster moulds and heat cured using a standard curing cycle for denture base acrylic. The samples produced were optically clear and essentially colourless, exhibiting an X-ray opacity similar to that of an equivalent thickness of aluminium at the wavelengths used for clinical diagnostic radiography.

The mechanical properties of the cured copolymers were not significantly different from unmodified polymethylmethacrylate (Trevalon).

### Typical mechanical properties:-

| | impact strength (J) | σ | flexural strength (MPa) | σ | Young's Modulus (GPa) | σ |
|---|---|---|---|---|---|---|
| Example 7 | 0.0280 | 0.0041 | 106 | 9 | 3.15 | 0.15 |
| Example 8 | 0.0260 | 0.0010 | 96 | 8 | 2.97 | 0.18 |
| Trevalon | 0.0273 | 0.0069 | 99 | 6 | 2.62 | 0.30 |

## Claims

1. An X-ray opaque composition which comprises an acrylate or methacrylate monomer having the general formula: 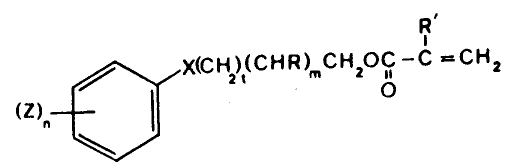
where X is -O- 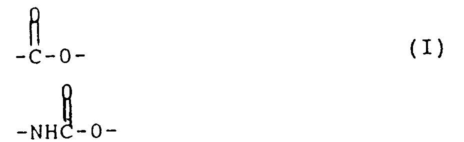
R is H, OH, CH₃, or 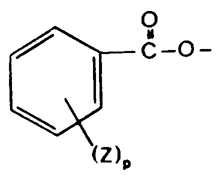
R' is H or CH₃
Z is Br or I, or a mixture of these substituents
m is 0 or 1
n is 3 to 5
p is 3 to 5
and t is 0 or 1,
with the proviso that when X is 0 then R is OH or 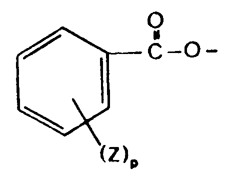
and m is 1; or a polymer or copolymer prepared therefrom.

2. A composition as claimed in claim 1 wherein the monomer is a methacrylate monomer which has the general formula:

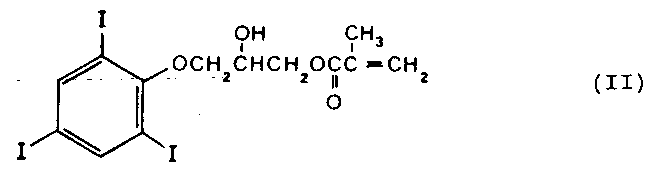

3. A composition as claimed in claim 1 wherein the monomer is a methacrylate monomer which has the general formula:

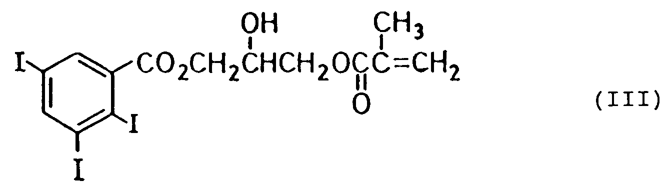

4. A composition as claimed in claim 1 wherein the monomer is a methacrylate monomer which has the general formula:

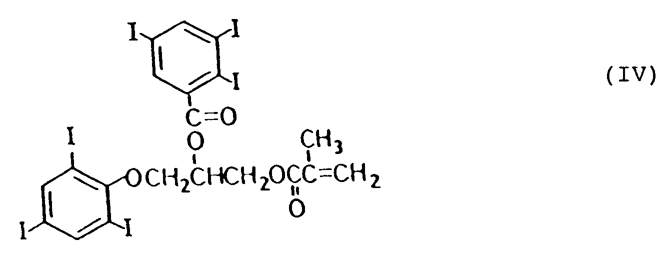

5. A composition as claimed in claim 1 wherein the monomer is a methacrylate monomer which has the general formula:

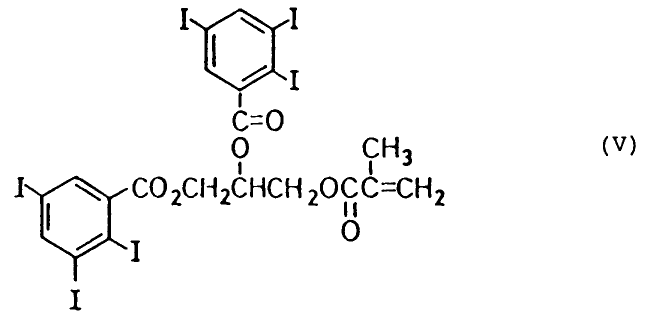

6. A composition as claimed in claim 1 wherein the monomer is a methacrylate monomer which has the general formula:

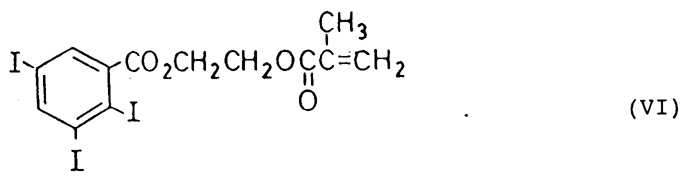

7. A composition as claimed in claim 1 wherein the monomer is a methacrylate monomer which has the general formula:

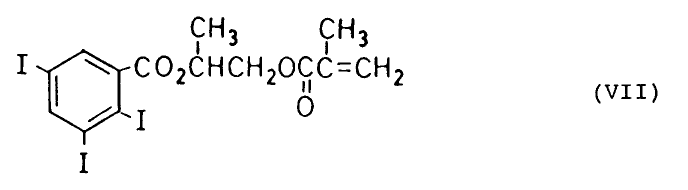

8. A composition as claimed in claim 1 which is a denture base composition which comprises from 5 to 50% by weight of the acrylate or methacrylate as defined in any one of claims 1 to 7, or from 5 to 50% by weight of a polymer prepared from the acrylate or methacrylate as defined in any one of claims 1 to 7.

9. A denture base composition as claimed in claim 8 which comprises the reaction product of polymethylmethacrylate and a methyl methacrylate monomer and wherein the acrylate or methacrylate as defined in any one of claims 1 to 7 is incorporated into the polymethylmethacrylate and/or into the methyl methacrylate monomer.

10. A denture base composition as claimed in claim 9 wherein the polymethylmethacrylate is in the form of beads.

11. A denture base composition as claimed in claim 10 wherein the beads have an average diameter in the range of from 40 to 250µm.

12. A composition as claimed in claim 1 which is an X-ray opaque bone cement which includes therein the acrylate or methacrylate as defined in any one of claims 1 to 7.

13. A composition as claimed in claim 1 wherein the monomer is X-ray opaque rubber which includes therein the acrylate or methacrylate as defined in any one of claims 1 to 7.

14. A composition as claimed in claim 1 which is in the form of X-ray opaque copolymer beads which comprise the acrylate or methacrylate monomer as defined in any one of claims 1 to 7 copolymerised with at least one comonomer.

15. A composition as claimed in claim 1 which is in the form of X-ray opaque teeth which include therein an acrylate or methacrylate as defined in any one of claims 1 to 7.

16. A composition as claimed in claim 1 which is an X-ray opaque polymer for biomedical applications which includes therein the acrylate or methacrylate as defined in any one of claims 1 to 7.

## Patentansprüche

1. Röntgenstrahl-opake Zusammensetzung, die ein Acrylat- oder Methacrylatmonomer der allgemeinen Formel umfaßt: 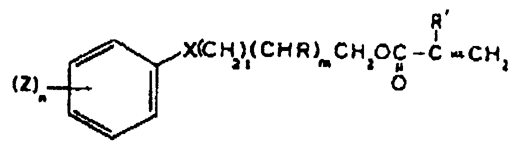
worin X -O- 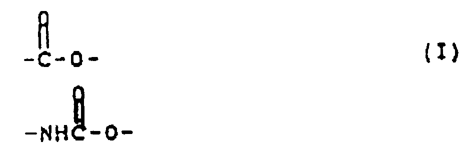 ist,
R H, OH, CH₃ oder 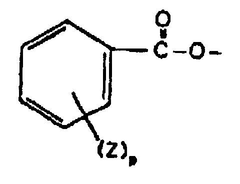 ist,
R' H oder CH₃ ist,
Z Br oder I oder ein Gemisch von diesen Substituenten ist,
m 0 oder 1 ist,
n 3 bis 5 ist,
p 3 bis 5 ist,
und t 0 oder 1 ist,
mit der Maßgabe, daß, wenn X 0 ist, dann R OH ist oder 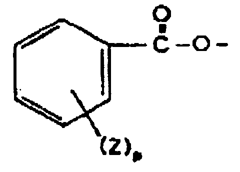
und m 1 ist; oder ein Polymer oder Copolymer hergestellt daraus.

2. Zusammensetzung nach Anspruch 1, worin das Monomer ein Methacrylatmonomer ist, welches die allgemaine Formal hat:

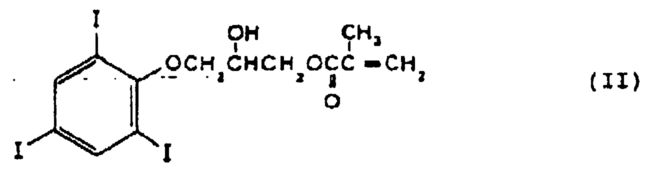

3. Zusammensetzung nach Anspruch 1, worin das Monomer ein Methacrylatmonomer ist, welches die allgemeine Formel hat:

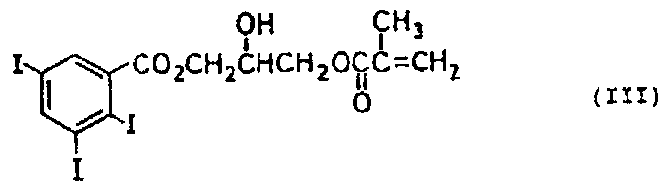

4. Zusammensetzung nach Anspruch 1, worin das Monomer ein Methacrylatmonomer ist, welches die allgemaine Formel hat:

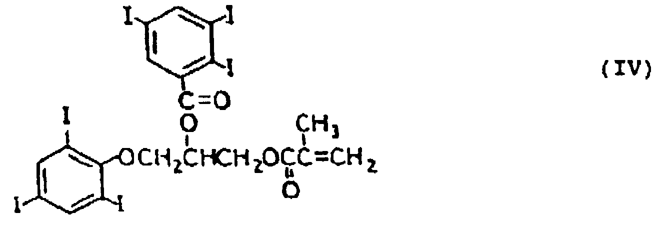

5. Zusammensetzung nach Anspruch 1, worin des Monomer ein Methacrylatmonomer ist, welches die allgemeine Formel hat:

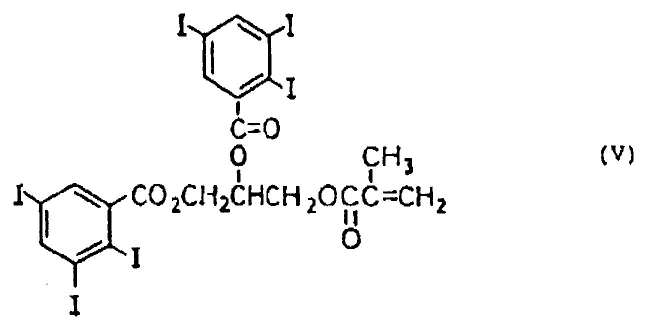

6. Zusammensetzung nach Anspruch 1, worin das Monomer ein Methacrylatmonomer ist, welches die allgemeine Formel hat:

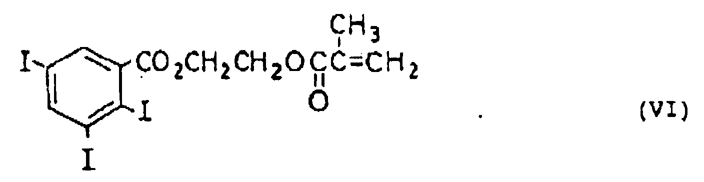

7. Zusammensetzung nach Anspruch 1, worin das Monomer ein Methacrylatmonomer ist, welches die allgemeine Formel hat:

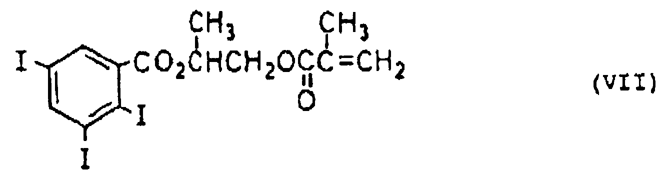

8. Zusammensetzung nach Anspruch 1, die eine Dentalzusammensetzung ist, welche von 5 bis 50 Gew.-% des Acrylats oder Methacrylats wie in einem der Ansprüche 1 bis 7 definiert, oder von 5 bis 50 Gew.-% eines Polymers, hergestellt aus dem Acrylat oder Methacrylat wie in einem der Ansprüche 1 bis 7 definiert, umfaßte.

9. Dentalzusammensetzung nach Anspruch 8, die das Reaktionsprodukt von Polymethylmethacrylat und einem Methylmethacrylatmonomer umfaßt, und worin das Acrylat oder Methacrylat Wie in einem der Ansprüche 1 bis 7 definiert in das Polymethylmethacrylat und/oder in das Methylmethacrylatmonomer eingebaut ist.

10. Dentalzusammensetzung nach Anspruch 9, worin das Polymethylmethacrylat in Form von Beads vorliegt.

11. Dentalzusammensetzung nach Anspruch 10, worin die Beads einen durchschnittlichen Durchmesser im Bereich von 40 bis 250 µm aufweisen.

12. Zusammensetzung nach Anspruch 1, die ein Röntgenstrahl-opaker Knochenzement ist, der das Acrylat oder Methacrylat wie in einem der Ansprüche 1 bis 7 definiert enthält.

13. Zusammensetzung nach Anspruch 1, worin das Monomer ein Röntgenstrahl-opaker Gummi ist, der das Acrylat oder Methacrylat wie in einem der Ansprüche 1 bis 7 definiert enthält.

14. Zusammensetzung nach Anspruch 1, die in Form Von Röntgenstrahlopaken Copolymerbeads vorliegt, welche das Acrylat- oder Methacrylatmonomer wie in einem der Ansprüche 1 bis 7 definiert copolymerisiert mit mindestens einem Comonomer umfassen.

15. Zusammensetzung nach Anspruch 1, die in Form von Röntgenstrahlopaken Zähnen vorliegt, welche ein Acrylat oder Methacrylat wie in einem der Ansprüche 1 bis 7 definiert enthalten.

16. Zusammensetzung nach Anspruch 1, die ein Röntoenstrahl-opakes Polymer für biomedizinische Anwendungen darstellt, welches Acrylat oder Methacrylat wie in einem der Ansprüche 1 bis 7 enthält.

## Revendications

1. Composition opaque aux rayons X, qui comprend un monomère acrylate ou méthacrylate répondant à la formule générale : 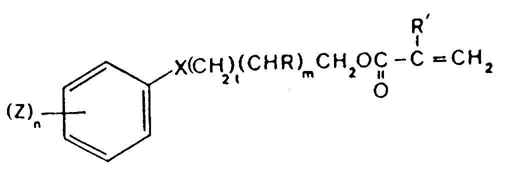
dans laquelle X représente -O-
un groupe 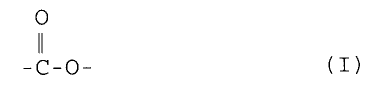 un groupe 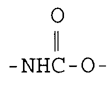
R représente H, un groupe OH, CH₃ ou 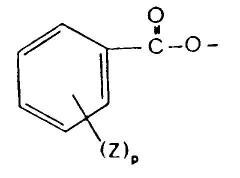
R' représente H ou un groupe CH₃
Z représente Br ou I, ou un mélange de ces substituants
m est égal à 0 ou 1
n a une valeur de 3 à 5
p a une valeur de 3 à 5
et t est égal à 0 ou 1,
sous réserve que, lorsque X représente O, alors R représente un groupe OH ou 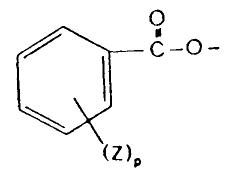
et m soit égal à 1 ; ou un polymère ou copolymère préparé à partir de ce monomère.

2. Composition suivant la revendication 1, dans laquelle le monomère est un monomère méthacrylate qui répond à la formule générale :

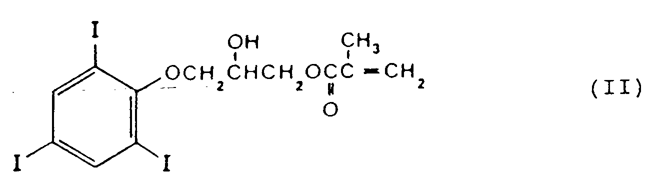

3. Composition suivant la revendication 1, dans laquelle le monomère est un monomère méthacrylate qui répond à la formule générale :

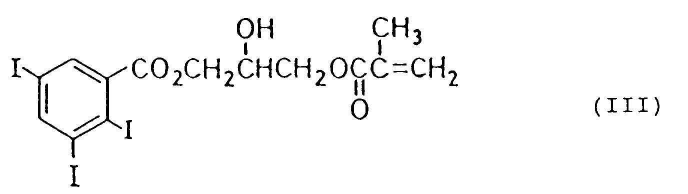

4. Composition suivant la revendication 1, dans laquelle le monomère est un monomère méthacrylate qui répond à la formule générale :

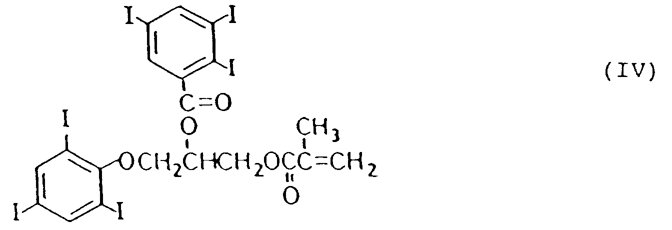

5. Composition suivant la revendication 1, dans laquelle le monomère est un monomère méthacrylate qui répond à la formule générale :

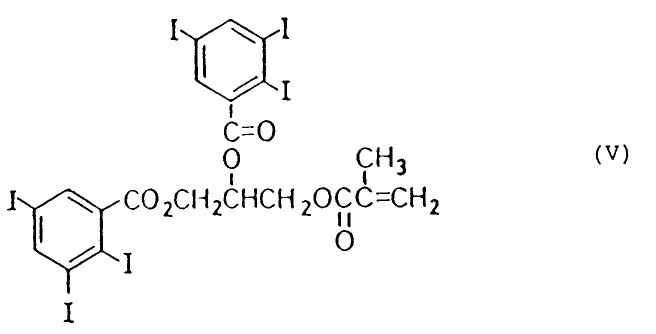

6. Composition suivant la revendication 1, dans laquelle le monomère est un monomère méthacrylate qui répond à la formule générale :

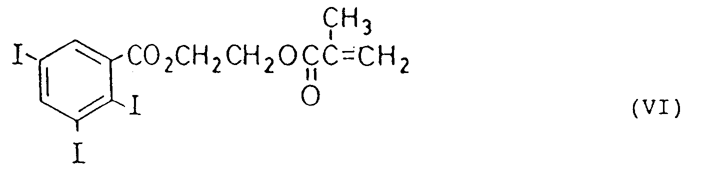

7. Composition suivant la revendication 1, dans laquelle le monomère est un monomère méthacrylate qui répond à la formule générale :

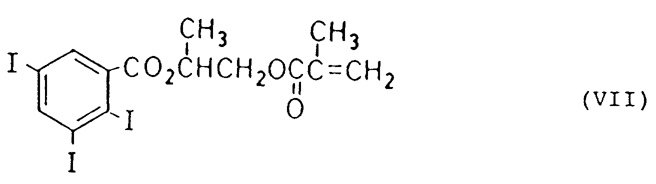

8. Composition suivant la revendication 1, qui est une composition de base de prothèse dentaire qui comprend 5 à 50 % en poids de l'acrylate ou du méthacrylate répondant à la définition suivant l'une quelconque des revendications 1 à 7, ou 5 à 50 % en poids d'un polymère préparé à partir de l'acrylate ou du méthacrylate répondant à la définition suivant l'une quelconque des revendications 1 à 7.

9. Composition de base de prothèse dentaire suivant la revendication 8, qui comprend le produit de réaction d'un polymère de méthacrylate de méthyle et d'un monomère méthacrylate de méthyle, et dans laquelle l'acrylate ou le méthacrylate répondant à la définition suivant l'une quelconque des revendications 1 à 7 est incorporé au polymère de méthacrylate de méthyle et/ou au monomère méthacrylate de méthyle.

10. Composition de base de prothèse dentaire suivant la revendication 9, dans laquelle le polymère de méthacrylate de méthyle est sous forme de billes.

11. Composition de base de prothèse dentaire suivant la revendication 10, dans laquelle les billes ont un diamètre moyen compris dans l'intervalle de 40 à 250 µm.

12. Composition suivant la revendication 1, qui est un ciment pour tissu osseux, opaque aux rayons X, qui renferme l'acrylate ou le méthacrylate répondant à la définition suivant l'une quelconque des revendications 1 à 7.

13. Composition suivant la revendication 1, dans laquelle le monomère consiste en un caoutchouc, opaque aux rayons X, qui renferme l'acrylate ou le méthacrylate répondant à la définition suivant l'une quelconque de revendications 1 à 7.

14. Composition suivant la revendication 1, qui est sous forme de billes de copolymère, opaques aux rayons X, qui comprend le monomère acrylate ou méthacrylate répondant à la définition suivant l'une quelconque des revendications 1 à 7 copolymérisé avec au moins un comonomère.

15. Composition suivant la revendication 1, qui est sous forme de dents, opaques aux rayons X qui renferme l'acrylate ou méthacrylate répondant à la définition suivant l'une quelconque des revendications 1 à 7.

16. Composition suivant la revendication 1, qui est un polymère, opaque aux rayons X, pour des applications biomédicales, qui renferme l'acrylate ou le méthacrylate répondant à la définition suivant l'une quelconque des revendications 1 à 7.
